# EUROPEAN PATENT APPLICATION

(11) **EP 4 527 935 A1**
(43) Date of publication of application: **26.03.2025**
(21) Application number: 23806459.6
(22) Date of filing: 19.05.2023
(51) Int. Cl.: C12P 1/02, C12N 1/22, C12N 1/14

(54) **METHOD FOR PRODUCING FUNGAL BIOCOMPOSITES USING LIGNOCELLULOSIC WASTE AND BYPRODUCTS, FUNGAL BIOCOMPOSITES, USE OF SAID BIOCOMPOSITES FOR PRODUCING CIVIL ENGINEERING AND SOUNDPROOFING ARTICLES, PACKAGINGS AND THE LIKE**

(30) Priority: 19.05.2022 BR 102022009786
(71) Applicant: Fungi Biotecnologia Ltda, 84017-220 Ponta Grossa (BR)
(72) Inventor: BITTENCOURT SYDNEY, Eduardo, CEP 84031-620 Ponta Grossa (BR); INAGAKI OSHIRO, Leandro, CEP 84010-280 Ponta Grossa (BR); CARLOS DE FRANCISCO, Antonio, CEP 84010-270 Ponta Grossa (BR)
(74) Representative: Pereira Garcia, João Luís
(86) International application number: PCT/BR2023/050151
(87) International publication number: WO 2023/220796

(57) **Abstract**

The present invention belongs to the biotechnology sector focused on the market of acoustics, civil construction, packaging, architecture and interior design, which refers, more specifically, to a process for the production of fungal biocomposites in two stages of growth, using as raw material lignocellulosic residues (bark, straw, bagasse, sawdust, etc.) from the agroindustry. The process aims to create fungal-based biocomposites to offer society sustainable consumer goods that are harmless to nature. This material has several chemical, physical and mechanical properties of interest that allow the development of solutions to replace materials of non-renewable origin in the areas of civil construction and acoustics (replacement of expanded polystyrene, glass wool and rock wool), protective packaging (replacement of plastics in general) and architecture/interior design (decorative items).

## Description

### Technological sector of invention

The present invention belongs to the Biotechnology sector focused on the acoustics, civil construction, packaging, architecture and interior design market, which refers, more specifically, to a process for the production of fungal biocomposites in three growth phases, using lignocellulosic waste (bark, straw, bagasse, sawdust, etc.) from the agroindustry as raw material.

The present invention also refers to fungal biocomposites and the use of these biocomposites to manufacture solutions to replace materials of non-renewable origin in the areas of civil construction and acoustics, protective packaging and architecture/interior design.

### State of the art

Modern chemical and nanotechnology materials have proven to be efficient for numerous applications, as they have precise specifications and varied properties. The attractiveness of producing these materials through biotechnology has been the subject of research around the world in order to develop new high-performance materials with low production costs and especially in a sustainable way. However, sometimes these materials continue to have high costs and have high difficulty in processing, production and even contradictorily with the production of aggressive residues (HANEEF et al., 2017, p. 1).

Because of this difficulty, the study of the creation of a biocomposite that does not need to undergo aggressive chemical processing to separate specific components becomes more necessary so that the concept of "green" material is more applicable. One strategy chosen is to use fungi, which grow superficially and in the intersections of the support material and form clusters of intertwined filaments (HANEEF et al., 2017, p. 1).

Fungi are the recyclers of biological materials from nature, which through the production of enzymes break down and dissolve various biological materials (NWE & FURUIKE & TAMURA, 2011, p. 189). Among the various existing types, the basidiomycetes belonging to the orders *Agaricales* and *Polyporales stand out,* in which the digestion mechanisms consist of the production of oxidative extracellular enzymes (laccases and peroxidases), which convert insoluble materials into soluble ones so that the cells of these fungi, called hyphae, can use them as nutrients (ABREU et al., 2007, p. 322; NWE & FURUIKE & TAMURA, 2011, p. 189).

The nutrients that the fungus absorbs are used for cell multiplication and consequently for colony growth, producing chitin, glucans, glutamates, d-mannose, d-galactose, fucoses, glycoproteins, glycopeptides, proteins, DNA, RNA, and other cellular materials (NWE & FURUIKE & TAMURA, 2011, p. 189).

The fungus permeates the substrate, through physical pressure and enzymatic secretion, absorbing nutrients from sources of carbon, nitrogen and other materials that are used for its growth, especially carbon, which is used for the formation of the cell wall, composed mainly of chitin, glucans and proteins (NWE & FURUIKE & TAMURA, 2011, p. 189; HANEEF et al., 2017, p. 2). Chitin, in addition to making up the cell wall of fungi, is also one of the main components of the exoskeleton of crustaceans and insects, which gives it the property of resistance and durability. (ROSS & WENNER & MOORLEGHEN, 2018, p. 25).

Biomaterials formed by mycelium (the name given to the tangle of hyphae) are realistic alternatives to petroleum-based materials, in addition to being obtained naturally, using little energy for production. They have good resistance and are resistant to water (ZELLER & ZOCHER, 2012, p. 52-54; HANEEF et al., 2017, p. 1).

Another factor to be considered in the production of fungal-based materials is the structures formed and complexity of structures formed by their hyphae. For example, fungi that have monomitic, dimitic and trimitic structures, differentiating mainly in the structures of the hyphae produced by them, in general, from monomythic to trimitic, increasing the complexity of the structures they can produce (JONES et. al., 2019, p.7).

Few companies in the world are working on the development of fungal biomaterials, such as Ecovative Design, which produces protective packaging material, construction materials, automotive materials, among others (ZELLER & ZOCHER, 2012, p. 54).

In 2017, the IBGE carried out an agricultural census on Plant Production and Extraction and Silviculture (PEVS), which allowed the updating of data on this production sector (IBGE - BRAZILIAN INSTITUTE OF GEOGRAPHY AND STATISTICS, 2017, p. 1). The data show that Brazil produces significant amounts of plant material that, in its processing, generate large amounts of by-products. According to the PEVS report, an income of R$ 19.1 billion was generated, of which 77.3% comes from forestry alone. The planted area is 9.8 million hectares, with the main regions being the South and Southeast of Brazil, which represent about 70.5% of this production (IBGE - BRAZILIAN INSTITUTE OF GEOGRAPHY AND STATISTICS, 2017, p. 1-3).

In 2017, there was a 5% growth in the participation of Silviculture compared to the previous year, and it can be mentioned that, of the entire replanting area, about 75% and 21% are Eucalyptus and Pinus, respectively. The southern region of Brazil produces 89% of the national Pinus, with the state of Paraná being responsible for 25% of the national production in 2021 (IBGE, 2017).

The usual destination of agricultural waste (biomass) is usually burning, and in the production of sugarcane about 95% of the bagasse is used as fuel for furnaces (CACURO & WALDMAN, 2015, p. 2156). New destinations for biomass generated by agricultural industries can be explored, in order to add value through a bioconversion of microorganisms, mainly from the Fungi kingdom (FIGUEIRO & GRACIOLLI, 2011, p. 924). In the case of cut wood production, 60% became waste, especially sawdust (also called saw dust). Sawdust is a waste of long biodegradability (accumulating in the environment) and low combustibility. Also, because it is light, it is easily carried by the wind and pollutes neighboring areas.

Thus, using fungi for growth in biomass (agricultural residues) to generate a biocomposite can become a solution based on circular economy, as proposed by Grimm (2018). This involves proposing a more appropriate destination for the growing biomass generated in agribusiness in Brazil (IBGE, 2019) and a reduction in gas emissions into the atmosphere by burning biomass, highlighted by Cacuro (2015), culminating in the generation of a biomaterial with useful properties for the market and capable of replacing materials traditionally produced from raw materials of fossil origin.

### Technical problem

The current modes of production and consumption are incompatible with the need to preserve the environment and people's quality of life. The excessive consumption of materials of non-renewable origin has caused environmental impacts that result in the loss of biodiversity, pollution of natural environments, decreased air quality, global warming, etc.

At the same time, Brazil is a country with an economy based on agribusiness. Agricultural production and food processing, as well as forestry, generate enormous amounts of waste (bark, straw, bagasse, cobs, sawdust, etc.) that can be used to produce materials of renewable origin. In grain production, for example, 2 tons of plant waste is generated for each ton of grain produced. This means that much more waste is produced than food.

The present invention presents a technology that uses these residues as a support and source of nutrients for the growth of a fungus. As it grows in the waste, the fungus acts as a natural glue aggregating particles and resulting in a biocomposite. This material has several chemical, physical and mechanical properties that have been analyzed in the laboratory. Among these properties, the following stand out: low density, flame resistance, moisture resistance, biodegradability, compostability in domestic conditions, mechanical resistance, low water footprint.

This set of characteristics allows the development of solutions for the replacement of materials of non-renewable origin in the areas of civil construction and acoustics (replacement of expanded polystyrene, glass wool and rock wool), protective packaging (replacement of plastics in general) and architecture/interior design (decorative items).

By taking advantage of these abundant lignocellulosic residues that are already produced, it would promote an optimization of the natural, financial, economic, human and time resources used in the production and processing of food.

The use of waste generated in agribusiness is still one of the great challenges that is faced not only at the national level, but worldwide. Some solutions that involve waste reuse processes can be highlighted. Patent document PI 0720758-1 describes a method of producing a composite material involving the formation of an inoculum that includes a pre-selected fungus, formation of a substrate mixture, and the inoculation of the fungus into the mixture. This method may also include placing the mixture in a casing prior to the addition of the inoculum, but it does not define the conditions and fungi that can be used and has failed to prove its technical effects.

The patent document US9410116 describes a method for growing organically derived building materials in the form of a moldable substrate that can be designed to serve a wide range of manufacturing and construction applications. In particular, the modalities consider a plurality of molded forms by fungi, preferably grown from the fungal inoculum and mechanically compacted at least once during the growth process, as well as the integration of the supporting members of the structure into the fungal structure. The solution provides a fungal substrate that can be easily and inexpensively molded and pre-processed to precise geometric specifications, according to the authors. However, the need to compact the material under high pressure (minimum 100PSI, preferably 500PSI) stands out. The physical and mechanical properties affected are not presented. In addition, layers of structural reinforcements are incorporated into building materials of organic origin to improve load bearing and other structural capabilities.

Another document is US20110268955 that demonstrates a method of manufacturing a molded part, including the formation of a liquid aggregate from a mixture of finely ground aggregate and a fluid. A mixture of a fungal inoculum and the liquid aggregate is formed. The nanoparticles are distributed homogeneously throughout the mixture and this is inserted into a cavity of the mold. Live mycelium is cultured to fill the mold cavity. This step in the process significantly undermines the scalability of the technology. After the mycelium has grown, the mold is heated to stop the growth. The addition of nanoparticles is described as an agent that improves characteristics, such as electrical conductivity, resilience, deflector capabilities, durability, rigidity, etc., but no data is demonstrated.

Furthermore, the WO2018014004 document reveals a method of forming fungal materials and fungal objects with characteristics of high-density, flexible and soft amorphous polymer, not being objects included in the present invention. The method comprises the stages of growth of a first fungal tissue in contact with a nutritive vehicle; supply of a porous material in contact with the first fungal tissue; directing the growth of said fungal tissue through said porous material so that a portion of said fungal tissue comprises a first fungal material having the first fungal hyphae; optionally incorporating composite material; targeting a change in the composition or growth pattern of at least some of the aforementioned early fungal hyphae; separation of at least one portion of the first fungal material from the said nutritive vehicle; and obtaining a second fungal material with second fungal hyphae. The object of this invention is a material composed of mycelium without the presence of the substrate used for its growth.

### News and objectives of the invention

This invention, in turn, is characterized as a technological platform for generating sustainable solutions from agricultural waste and fungi. The fungus growth process comprises three stages, the first being carried out in cultivation bags, the second in molds (final product acquires the shape of the mold) and the third outside the molds (finishing). In the production process, it is understood that to improve the characteristics of the resulting material, supplementation can be added at the stage in which the materials are packaged in the molds.

The three-stage production system allows for greater dynamics in production, separating the stages of fungal growth from the formation of a final product. It also results in the reduction of productivity losses due to the possibility of better controlling the conditions of fungus cultivation (separate rooms for fungus production and cultivation of the final product inside and outside molds), reduction in contamination, ease in scaling the process, improvement of the characteristics of the material resulting by supplementation. The separation of the process phases promoted a fourfold reduction in the contamination rate when compared to the traditional single-phase method.

Finding sustainable options for replacing materials from non-renewable sources is a goal these days. The technology presented in this document aims to promote the transformation of agricultural and forestry waste into an innovative and sustainable material, using clean technology, capable of generating carbon credits and recognized by the SESI SDG 2021 seal.

Several variables can be altered for mycelial growth in the chosen substrate (such as supplementation in the substrate, variations in humidity, temperature, CO2 concentration and luminosity in the growth environment, pre-processing and post-processing), which can imply changes in the cell growth process of the fungus and in the compounds it produces. Any of these changes can result in different properties in the material, and may have properties similar to those of thermoplastics, insect exoskeletons, animal leather, foams, construction materials, food packaging and high-performance materials (MCINTYRE et al., 2015, p. 3; BETTS & TUDRYN & HART (2016), 6; ROSS, 2016, p. 13; ROSS & WENNER & MOORLEGHEN, 2018, p. 26).

### Description of the attached drawings

In order for this invention to be fully understood and put into practice by any technician in this technological sector, it will be described in a clear, concise and sufficient manner, based on the attached drawings, which illustrate and support it, listed below:
Figure 1 represents the flowchart of the stages of the process object of this invention;
Figure 2 represents examples of products obtained by the process object of this invention;
Figure 3 represents examples of manufactured acoustic panels;
Figure 4 represents the sound absorption graph for each third-octave frequency range of 18mm thick acoustic panels;
Figure 5 represents the sound absorption evaluation graph in an impedance tube with different formulations of phase III;
Figure 6 represents the evaluation of the flammability of the biocomposites produced with buckwheat husks;
Figure 7 represents a plate where a flame was applied.

### Detailed description of the invention

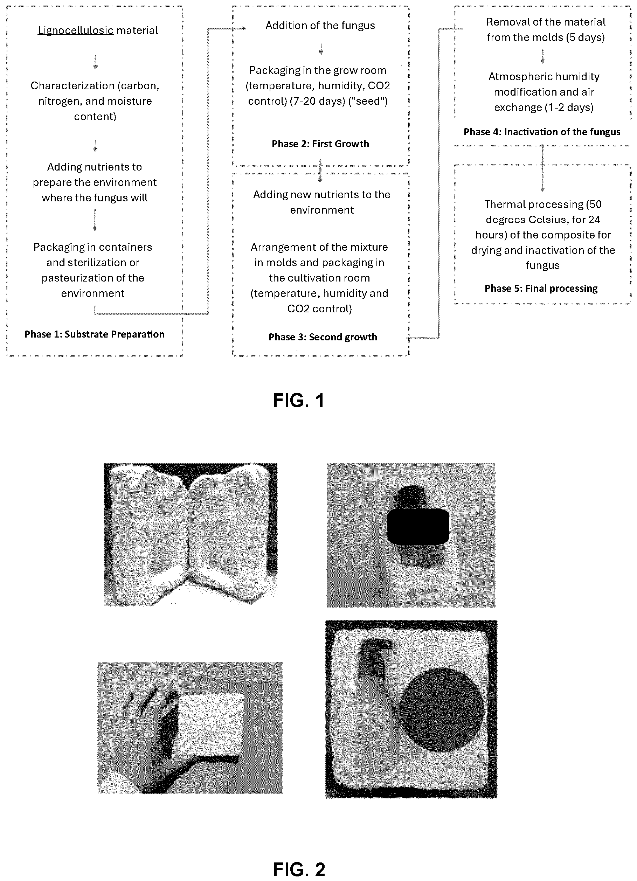

Finding sustainable options for replacing materials from non-renewable sources is a goal these days. Adding the ideals of biotechnology and the wide range of properties of fungal growth, this invention aims to create fungal-based biocomposites to offer society sustainable consumer goods that are harmless to nature. The process of this invention comprises 5 phases:
I) Substrate preparation; II) First growth; III) Second growth; IV) Third growth; and V) Final processing, as shown in Figure 1 and discussed below.

### PHASE I - SUBSTRATE PREPARATION

### Lignocellulosic material and characterization

Several lignocellulosic materials can be used for the production of fungal biocomposites. Mainly sawdust and harvest (wood industry waste), buckwheat husks (buckwheat), rice husks, corn residue, soybean residue and sugarcane bagasse were used. Each lignocellulosic material has a specific composition, which must be adjusted in order to promote the growth of the fungus (Table 1).

**Table 1. Carbon/nitrogen ratio of lignocellulosic materials**

| **Lignocellulosic Material** | **%N** | **Relation C/N** | **%C** |
|---|---|---|---|
| **Cotton: Seed Residue** | **1,06** | **50,00** | **53,00** |
| **Cotton: burnt seed** | **4,58** | **12,00** | **54,96** |
| **Mulberry: leaves** | **3,77** | **12,00** | **45,24** |
| **Rice: husk** | **0,78** | **39,00** | **30,42** |
| **Rice: straw** | **0,78** | **39,00** | **30,42** |
| **Oats: husk** | **0,75** | **63,00** | **47,25** |
| **Oats: straw** | **0,66** | **72,00** | **47,52** |
| **Sugarcane bagasse** | **1,07** | **37,00** | **39,59** |
| **Banana: leaves** | **2,58** | **19,00** | **49,02** |
| **Banana: stalk and bunch** | **0,77** | **61,00** | **46,97** |
| **Coffee grounds** | **2,30** | **22,00** | **50,60** |
| **Cocoa: peel and fruits** | **1,28** | **38,00** | **48,64** |
| **Cocoa: film** | **3,24** | **16,00** | **51,84** |
| **Coffee: peels** | **0,86** | **34,93** | **30,04** |
| **Coffee: straw** | **0,62** | **83,44** | **51,73** |
| **Coffee: denatured seed** | **3,27** | **16,00** | **52,32** |
| **Lemon balm** | **0,82** | **71,76** | **58,84** |
| **Fat grass** | **0,63** | **81,00** | **51,03** |
| **Guinea grass** | **1,49** | **33,00** | **49,17** |
| **Jaguará grass** | **0,79** | **64,00** | **50,56** |
| **Rice husk** | **0,78** | **39,00** | **30,42** |
| **Buckwheat husk** | **2,02** | **15,87** | **32,00** |
| **Barley: pomace** | **5,13** | **10,00** | **51,30** |
| **Barley: husks** | **0,56** | **85,00** | **47,60** |
| **Barley: straws** | **0,75** | **63,00** | **47,25** |
| **Powder leather** | **8,74** | **5,01** | **43,75** |
| **Poultry manure** | **2,76** | **10,51** | **29,01** |
| **Cattle manure** | **1,67** | **32,00** | **53,44** |
| **Equine manure** | **1,67** | **15,27** | **25,50** |
| **Sheep manure** | **1,44** | **32,00** | **46,08** |
| **Pig manure** | **1,86** | **15,86** | **29,50** |
| **Eucalyptus: waste** | **2,83** | **15,00** | **42,45** |
| **Yeast extract** | **10,00** | **3,95** | **39,53** |
| **Wheat bran** | **0,45** | **5,96** | **2,67** |
| **Wheat flour** | **1,60** | **22,26** | **35,62** |
| **Potato starch** | **1,10** | **34,27** | **37,84** |
| **Pigeon peas: straw** | **1,81** | **29,00** | **52,49** |
| **Pigeon peas: seed** | **3,64** | **14,97** | **54,50** |
| **Bean: straws** | **1,63** | **32,00** | **52,16** |
| **Smoking: waste** | **2,17** | **18,00** | **39,06** |
| **Bahia grass** | **1,39** | **36,00** | **50,04** |
| **Silk Grass** | **1,62** | **31,00** | **50,22** |
| **Orange: pomace** | **0,71** | **18,00** | **12,78** |
| **Cassava: leaves** | **4,35** | **12,00** | **52,20** |
| **Corn: straws** | **0,48** | **112,00** | **53,76** |
| **Corn: cob** | **0,52** | **101,00** | **52,52** |
| **Chicken feathers** | **13,55** | **4,00** | **54,20** |
| **Fern** | **0,49** | **109,00** | **53,41** |
| **Dried blood** | **11,80** | **4,00** | **47,20** |
| **Sawdust** | **0,06** | **865,00** | **51,90** |
| **Leaf litter** | **0,96** | **17,00** | **16,32** |
| **Cotton pie** | **5,68** | **9,00** | **51,12** |

The main parameters used for fungal growth in the substrates are: moisture and the ratio between carbon and nitrogen content. Moisture is usually measured in the laboratory (drying in an oven and measuring the weight difference), while the C/N ratio is extracted from the scientific literature or analyzed.

Lignocellulosic materials can be crushed and screened for size standardization (particle size). Different particle sizes and characteristics give different properties to the final product (especially with regard to density, hardness and mechanical resistance. In the case of density, for example, materials produced with pine sawdust in the MESH 10 size have 0.325g/cm², while in MESH 5/8 the density is 0.133g/cm³. Particles of lignocellulosic materials can be in two main forms: fiber and granule, as well as a mixture depending on the mixture used.

### Addition of nutrients for formulation preparation

According to the composition of the lignocellulosic material, and knowing the preferred conditions of the fungi used *(Ganoderma lucidum, Ganoderma neo-japonicium, Ganoderma resinaceum, Trametes versicolor and Pleurotus ostreatus),* in the optimization of growth, it is necessary to add nutrients. The nutrients usually added are: wheat bran (which can be another serving to adjust the amount of nitrogen), calcium carbonate (pH control), shell limestone (source of calcium) and water (in order to adjust the gravimetric content to 45 to 70% preferably, depending on the absorption capacity of the substrate).

For example, to prepare a formulation based on buckwheat husks, the following are used:
- 78% buckwheat hull (whole or crushed);
- 20% wheat bran;
- 1% calcium carbonate;
- 1% shell limestone.

To prepare a formulation based on harvesting or sawdust (or a mixture of both in different proportions), the following are done:
- 88% of scoop or sawdust;
- 10% wheat bran;
- 1% calcium carbonate;
- 1% shell limestone.

Coffee grounds:
- 99% coffee grounds;
- 1% calcium carbonate.

Malt pomace:
- 50% malt bagasse;
- 49% wheat bran;
- 1% calcium carbonate.

Corn husks (usually crushed):
- 86% corn husk;
- 10% wheat bran;
- 2% calcium carbonate;
- 2% shell limestone.

It should be noted that a mixture of these materials can be used. Each lignocellulosic material and its respective mixtures provide different properties to the final product. From phase II to phase V the processing does not change, regardless of the lignocellulosic material used.

Fungi grow in the so-called lignocellulosic materials, in various compositions and the cases mentioned above are formulations that have an exemplification character.

In the present case, it is known that materials with smaller granulometry (smaller particles, with MESH ranging from 3/8 to 30) provide greater mechanical resistance to the material, and they should be used mainly in civil construction and packaging. When larger particle materials are used (such as MESH 5/5 sawdust or sawdust, for example, or whole buckwheat husk), the material is much more flexible (less mechanical resistance) and has better acoustic absorption - mainly applied in the manufacture of acoustic panels and coatings.

### Packaging in containers and sterilization or pasteurization

The mixture, prepared, is packed in containers, which can be plastic, glass or metal. It is followed by the sterilization stage at 121°C for 90 minutes (which may vary proportionally with the volume of the container and the volumetric capacity of the autoclave). Alternatively, pasteurization can be carried out at 70 to 80°C for 10 to 12 hours. Before the next step, the substrate must be cooled to a maximum temperature of 35 °C.

### PHASE II - FIRST GROWTH

In a microbiologically controlled environment (as aseptic as possible), the fungus is added to the mixture. The fungi used are *Ganoderma lucidum, Ganoderma neo-japonicium, Trametes versicolor* and *Pleurotus ostreatus.* Highlighting the white rot fungi that have trimitic hyphal structures.

The inoculated substrates are stored in cultivation rooms with controlled temperature and humidity. The temperature is ideally 25 °C (it can vary from 20 to 33 °C) and the relative humidity preferably at 70%, and can vary from 60 to 80%. At this stage, the flow and exchange of air with the environment is also controlled in order to accelerate the growth of the fungus. By keeping the CO2 concentration between 500 and 3,000 ppm, it causes the mycelium to have an optimized growth.

### PHASE III - SECOND GROWTH

At the end of the Phase II growth, which lasts between 3 and 20 days, and in preferred implementations of 3 to 7 days, depending on the lignocellulosic material and the fungal strain used, a first block of fungal biocomposite is formed. This block is broken (crushed into small particles) and new nutrients are added to the mixture.

In this step, in a first preferred implementation, a mixture composed of the following is added to the crushed biocomposite, in relation to the wet weight of 5 to 15%, in relation to the wet weight of 5 to 15%.
- 74% wheat flour;
- 7.4% gypsum;
- 7.4% shell limestone;
- 7.4% cellulose;
- 3.8% lignin.

And, in a second preferred implementation, a mixture composed of the following is added to the crushed biocomposite, in relation to the wet weight of 3 to 9%, consisting of:
- 74% wheat flour;
- 7.4% gypsum;
- 7.4% shell limestone;
- 7.4% cellulose.

Supplementation can be carried out by adding a single component alone and/or a combination of them, not limited to those mentioned above, as long as it aims to improve the structural characteristics of the mycelium and promote growth.

The material is then packaged in shapes (molds) with the format that the final product is desired to have. These molds can be made of the most diverse materials, as long as they are inert to water (preferably they should not absorb moisture). The molds with the material are stored in cultivation rooms, with a temperature of 25°C (which can vary from 20 to 33°C). In a preferred realization of the invention, the humidity can vary between 60 and 80%, preferably 75%. In a second preferred realization of the invention, the humidity can vary between 55 and 100%, preferably being 96%. . The flux of CO2 can vary from 500-50,000ppm depending on the fungus, preferably from 1,000 to 30,000ppm. The material completes this second growth phase in 1 to 4 days, preferably 2 days.

### PHASE IV - THIRD GROWTH

The material is removed from the mold and kept in an environment with a controlled temperature of 25°C (which can vary from 20 to 33°C). The humidity is changed to 95% preferentially (which can be 75 to 95%) and the CO2 content is reduced (allowing more gas exchange, lowering the CO2 range to 500-10,000ppm).

The material is kept in this environment for 1 to 2 days.

### PHASE V - FINAL PROCESSING

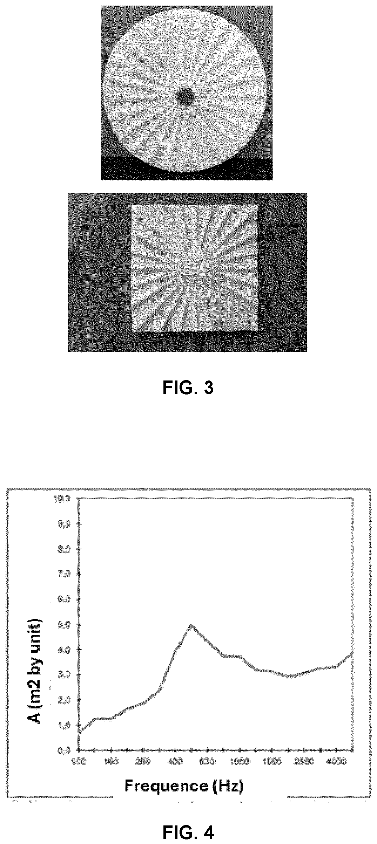

At the end of growth, the fungal biocomposite is heated to at least 24°C in a preferred realization and at least 50°C in a second preferred implementation, reaching a maximum temperature of 240°C, preferably 50°C for at least 24 hours for inactivation (death) of the fungus and drying, so as not to proliferate other contaminants and decomposing agents (the drying time of the product depends on its size). As a parameter, the time required for the product to reach 40% of the initial weight value after the procedure is used). Examples of end products are shown in Figures 2 and 3.

### Production of acoustic boards using wood sawdust as raw material

The production of acoustic boards using wood sawdust begins with the standardization of the granulometry of the raw material. The grain size can be adjusted according to the desired mechanical properties (physical strength, for example) and density. In this case, Pinus sawdust was used in granulometry ranging from 0-2 mm of particle size. Standardized sawdust by sieving is used in the composition of the substrate for solid fermentation.
- 88% sawdust;
- 10% wheat bran;
- 1% calcium carbonate;
- 1% shell limestone.

Water is added to the nutrient mixture in order to obtain moisture between 30 and 80%, preferably 65%. The substrate is packaged in containers that can be sterilized or pasteurized. An opening is made in these containers so that gas exchange is allowed. After sterilization, the desired fungal species, in this case *Ganoderma lucidum, is added* in a ratio of 1 to 25% (w/w). The cultivation container is stored in an environment with a controlled temperature of 25°C and humidity of 80%, and air exchanges are carried out to control the concentration of CO2 in the environment (25 air changes per hour).

After 4 to 15 days, preferably 8 days of cultivation, the container is opened and the cultivation is mechanically crushed. 15% wheat flour, 1% lignin or glucose or sucrose or cellulose are added; 1% Gypsum and 1% Shell limestone. Then, the mixture is packed in molds in the desired shape. All manipulation for shredding the substrate, mixing and packaging in molds must be carried out in a controlled environment (but not aseptic - an environment without air circulation, on clean surfaces and using good microbiological handling practices such as the use of gloves, masks, etc.). In the case of the square plate, the mold has dimensions 33 cm x 33 cm x 2.5 cm.

The molds are then placed in a new environment with a temperature of 25°C and humidity of 80% controlled, and air exchanges are carried out to control the concentration of CO2 in the environment (20 air exchanges per hour). At the end of 1 to 4 days, preferably 2 days, the product is removed from the mold and left in the same environment for another 1 to 4 days, preferably 2 days. In the last step, the product is dehydrated using an oven/oven at 50°C for 6 to 24 hours or at room temperature until it reaches constant weight.

An evaluation of the acoustic absorption capacity was carried out according to ISO 354:2003 Acoustics - Measurement of sound absorption in a reverberation room, with assembly based on the general guidelines of type A, Annex B of ISO 354:2003, reaching a mean absorption coefficient (αw) of 0.40, NRC = 0.35 and SAA = 0.33, as shown in Figure 4.

The material demonstrated a superior acoustic absorption capacity to common materials used indoors, proving the action of absorbing sound waves. The sound absorption capacity can be optimized by increasing the thickness of the plate or depending on the form of installation. The acoustic evaluation was carried out by mimicking the installation of the boards by gluing them to a masonry wall. In this case, the acoustic absorption is done exclusively by the front face of the material. In the case of installation where the board is not glued to the wall, allowing a space to form between it and the wall, the absorption rate is significantly increased by exploiting the two sides of the material. The choice of acoustic materials depends not only on αw, NRC and SAA, but on the absorption capacity at specific wavelengths, which depend on the type of environment in which they will be installed. In this way, the acoustic plates described in this document are characterized as unique and innovative materials. The material of this invention has, for example, NRC very similar to that of commercial products produced from polyurethane polyester foam with greater thickness (material with 20mm has NRC = 0.36 and with 40mm has NRC = 0.39).

### Evaluation of sound absorption in an impedance tube with different formulations of phase III

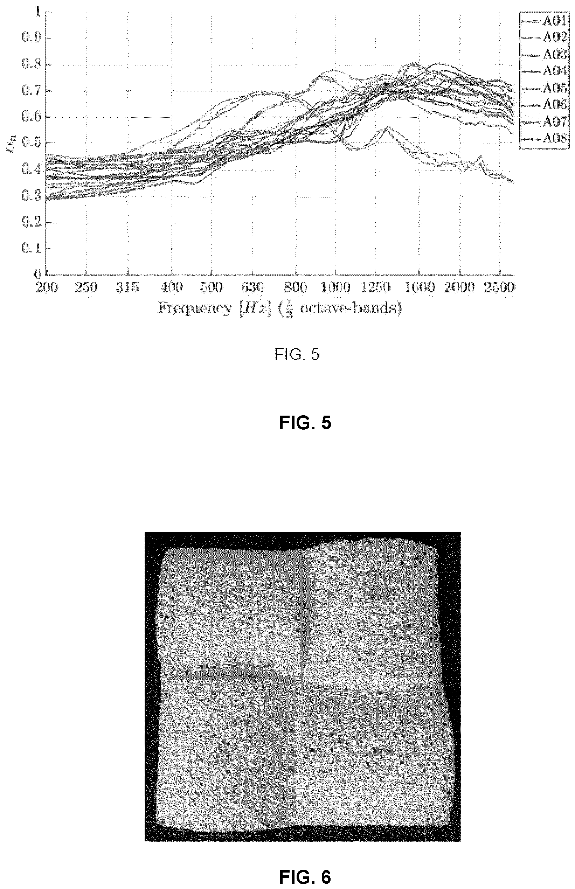

The effect of the formulation of the substrate containing wood sawdust on the acoustic absorption capacity of the impedance tube material was evaluated. Production followed Phase II of the process (first growth phase), but modifications were made to the components added in Phase III (second growth phase). Sample A01 did not receive any nutrient supplementation, A02 only wheat flour (15%); A03 flour, limestone and gypsum (15%, 1%, 1%, respectively); A04 flour, limestone, gypsum and cellulose (15%, 1%, 1%, 1%, respectively); A05-A08 flour, limestone, gypsum, cellulose and lignin (15%, 1%, 1%, 1%, 1%, respectively), all the tests were performed in triplicates, as shown in Figure 5.

It was observed that the acoustic capacity of the tests from A03 to A08 shows an improvement in the regularity of the material for absorption of waves at higher frequencies, and the tests from A04 to A08 show a slight improvement in the acoustic capacity of the material, validating the importance of the correct supplementation of Phase III. presents an innovation and proves the versatility of this technology.

### Evaluation of the flammability of biocomposite with buckwheat hull

The production of slabs using buckwheat husk begins with the addition of components and mixture in the following composition for fermentation in solid state:
- 78% buckwheat hull (whole or crushed);
- 20% wheat bran;
- 1% calcium carbonate;
- 1% shell limestone.

Depending on this, buckwheat husk can be used crushed and also standardized with varying sizes according to the desired characteristics of the resulting material.

To the nutrient mixture, water is added in order to obtain humidity between 30-50%. The substrate is packaged in containers that can be sterilized or pasteurized. An opening is made in these containers in such a way that gas exchange is allowed and that keeps the environment free of microorganisms of contaminants. After sterilization, the desired fungal species, in this case *Ganoderma lucidum,* is added at a ratio of 1-25% (w/w). The cultivation container is stored in an environment with a controlled temperature of 25°C and humidity of 80%, and air exchanges are carried out to control the concentration of CO2 in the environment (25 air changes per hour). After 4 to 15 days, preferably 8 days of cultivation, the container is opened and the cultivation is mechanically crushed. 15% wheat flour, 1% lignin or glucose or sucrose or cellulose were added; 1% Gypsum and 1% Shell limestone. Then, the mixture was packed in molds in the desired shape. All handling for shredding the substrate, mixing and packaging in molds must be carried out in a controlled environment (but not aseptic - an environment without air circulation, on clean surfaces and using good microbiological handling practices such as the use of gloves, masks, etc.). In the case of the square plate, the mold has dimensions 33 cm x 33 cm x 2.5 cm.

The molds are then placed in a new environment with a temperature of 25°C and humidity of 80% controlled, and air exchanges are carried out to control the concentration of CO2 in the environment (20 air exchanges per hour). At the end of 1 to 4 days, preferably 2 days, the product is removed from the mold and left in the same environment for another 1 to 4 days, preferably 2 days. In the last step, the product is dehydrated using an oven/oven at 50°C for 6 to 24 hours or at room temperature until it reaches constant weight, as shown in Figure 6.

After drying, a flammability study of the material "UL 94 - Vertical burn test" and "UL 94 - Horizontal burn test" was carried out, in which the material was cut to the dimensions (127 x 13 x 3.2 mm) specified in the test in question, culminating in the following results:

| **Body of the test** | **T1(s) - Propagation time after removal of the flame (1st application)** | **T2(s) - Propagation time after removal of the flame (2nd application)** | **Σ( T1+T2) (s)** | **Cotton burning** | **Classification *- Method Vertical Burning*** |
|---|---|---|---|---|---|
| CP1 | 4 | 5 | 9 | ( ) Yes | V-0 |
| | | | | (x) no | |
| CP2 | 3 | 4 | 7 | ( ) Yes | V-0 |
| | | | | (x) no | |
| CP3 | 5 | 6 | 11 | ( ) Yes | V-1 |
| | | | | (x) no | |
| CP4 | 4 | 4 | 8 | ( ) Yes | V-0 |
| | | | | (x) no | |
| CP5 | 4 | 5 | 9 | ( ) Yes | V-0 |
| | | | | (x) no | |

The material in question has the ability to self-extinguish the flame with one of the best classifications applied by the method in question.

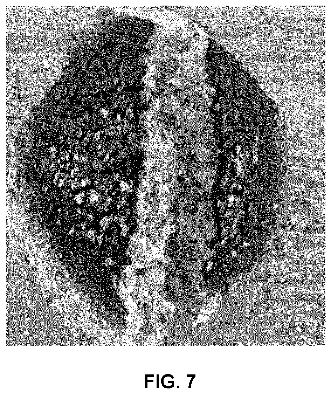

In order to apply this scale test, a plate with a dimension of approximately 10 x 10 cm was cut out and a direct flame (Figure 7) from a butane-fueled torch was applied at a distance of 5 cm from the plate for one minute. Soon after the flame was turned off, the material ceased burning, becoming incandescent for 3 seconds.

The material, in addition to being self-extinguishing, has excellent flame resistance, preventing it from burning the material internally. While it was validated in the laboratory on a small scale, tests were carried out in accordance with ASTM E662: 2021 and ABNT NBR 9442:2019, with Ip=1.71 and Dm=390.87 being determined, categorizing the material with Class II-A according to the classification according to ABNT NBR 16626:2017 and IT No. 10:2019

### Evaluation of the biodegradability of biocomposite with sawdust

Biodegradability evaluation was carried out according to the 301B-OECD standard. The assay was conducted using a sample of effluent from a treatment plant that contained 1.60x107 CFU/mL. The carbon content in the evaluated material was equal to 45.05 and the amount of sample used was 0.074g/3L, and the test was conducted at 21.5 ± 0.5°C for 28 days. The assay was considered validated because the difference between the extremes in the duplicates was less than 20%. The biodegradable standard showed biodegradation of 60% of CO2 evolution in 10 days from the moment when 10% of CO2 evolution was observed and did not exceed the maximum period of 14 days, and there was no inhibition of the microorganisms used since the treatment inhibition reached 25% of CO2 release in 14 days.

According to the methodology used and under the conditions of the assay, the test item showed 100% biodegradation within the period of 28 days and reached the criterion of rapid biodegradation (60% ThCO2 and compliance with the 10-day window). Thus, the item was considered quickly biodegradable.

Biodegradability tests were also carried out under domestic conditions and in different environments. Waste plates produced from this invention were sent for analysis of the parameters of biological contaminants and heavy metals according to MAPA - IN SDA 27, June 2006. The biological contaminants evaluated were viable helminth eggs, thermotolerant coliforms and Salmonella, which presented quantities below the limits required by MAPA for fertilizer production. Regarding heavy metals, Arsenic, Cadmium, Lead, Chromium, Chromium VI, Mercury, Nickel and Selenium were evaluated, all of which presented concentrations below the detection limit of the equipment. Thus, these results are in accordance with those required as a good soil conditioner after composting process as required (MAPA - IN SDA 27, June 2006).

Then, the residues of the invention plates were separated and conducted under different conditions and/or natural environment using 10-gram samples. The materials were packed in plastic trays and subjected to 6 different conditions:
- Treatment 1: Material of this invention *in natura* packed on a plastic tray, without the presence of water, soil or sand;
- Treatment 2: Material of this invention *in natura* packed on a plastic tray, moistened with 20 mL of water 1 time a week;
- Treatment 3: Material of this invention *in natura* packed on a plastic tray filled with water (constant 2000 mL);
- Treatment 4: Material of this invention *in natura* packed on a plastic tray filled with water with NaCl (constant 2000 mL);
- Treatment 5: Material of this invention *in natura* packed on a plastic tray on the sand, receiving irrigation of 500 mL of water 1 time a week;
- Treatment 6: Material of this invention *in natura* placed in a plastic tray on the ground, receiving irrigation of 500 mL of water 1 time a week.

The treatments were conducted for 106 days, under room temperature (see table below). The tests performed in a liquid environment were conducted without agitation. At the end of the test, all materials were sent for analysis of chemical, physical and biological parameters. Therefore, this study also aimed to determine the nutritional behavior, physical, chemical and biological characteristics of the residues of the invention plates as a function of different biodegradation processes, which may or may not be used as a soil conditioner.

**Table: pH values, Humidity; Total Organic Carbon, Conductivity, Organic Matter, Cation Exchange Capacity (CEC), Water Retention Capacity (CRA) and Volatile Solids, from the different composting processes in the invention's board waste: in natura; wet material; sand; soil; H20; H20+NaCl, at the end of 106 from the beginning of the treatments**

| **TREATMENTS** | | | | | | |
|---|---|---|---|---|---|---|
| | **T1** | **T2** | **T3** | **T4** | **T5** | **T6** |
| **Elements** | Micronutrients (g/dm³) | | | | | |
| **pH** | 4.87 | 8,36 | 8,04 | 7,99 | 7,92 | 7,75 |
| **Humidity (at** 65°C) (%) | 7,87 | 64,00 | ... | ... | 25,19 | 61,92 |
| Totat Organic Carbon (%) | 40,05 | 44,14 | 0.30 | 0,15 | 22,04 | 40.17 |
| **Conductivity µS/cm** | 2,41 | 581,00 | 123,20 | 861,00 | 361.00 | 202,00 |
| **Organic Matter (%)** | 89,71 | 86,99 | *** | *** | 16,37 | 71,19 |
| **CTC (mmol/L)** | 315,53 | 184,48 | *** | *** | *** | 462,69 |
| **CRA (mm) (%)** | 323.73 | 501,39 | *** | ... | *** | 437,15 |
| **Volatile solids (%)** | *** | *** | 70,77 | 42,39 | *** | *** |

It was observed that the material in the T1 treatment did not present any visual alteration in terms of degradation of structures, color alteration or even growth of opportunistic microorganisms. This result shows that the material was stable, preserving the characteristics during the evaluated period (106 days). It was observed that the other types of treatment imposed, when compared with the initial treatment (in natura T1), resulted in reductions in the nutritional contents of Nitrogen, Total Phosphorus (P), Total Phosphorus (P2O5), Total Potassium (K), Total Potassium (K2O), Sulfur, Calcium and Magnesium. In the soil sample, the increase in the levels of Nitrogen and Phosphorus are a consequence of the mixture of the Material of this invention with the soil sample, which made it impossible to completely separate it for the performance of the nutrient analysis. In relation to micronutrients (Boron, Iron, Zinc, Copper and Manganese), a significant reduction was also observed in all parameters evaluated, with the exception of Boron (B) whose behavior was inverse for all the treatments imposed.

During composting, a significant increase was noticed for all treatments for pH and conductivity compared to the *in natura treatment.* There was also a 500% increase in moisture content in treatments 5 and 6, which strengthens the potential of this material as an important component of the soil to increase water retention capacity (CRA). By observing the higher values of 200 mmolc.kg-1 for CEC and 60% for CRA, in the T6 treatment, and by comparing with the information described in the normative instruction MAPA - IN DAS N° 35, of July 4, 2006, it is observed that the residues of the invention plates, when incorporated with the soil, can improve the conditions of the soil, physical, chemical and biological characteristics, functioning as a good class A soil conditioner. These results demonstrate the intense interaction of the plates of the invention with the soil, favoring the composting process.

It is important to emphasize that the figures and description made do not have the power to limit the forms of execution of the inventive concept proposed here, but rather to illustrate and make understandable the conceptual innovations revealed in this solution. Thus, the descriptions and images must be interpreted in an illustrative and non-limiting way, and there may be other equivalent or analogous ways of implementing the inventive concept revealed here and that do not escape the spectrum of protection outlined in the proposed solution.

## Claims

1. PRODUCTION PROCESS OF FUNGAL BIOCOMPOSITES USING LIGNOCELLULOSIC RESIDUES AND BY-PRODUCTS **characterized by** comprising the following steps:
I. SUBSTRATE PREPARATION
a) The selected lignocellulosic materials are separated from the group consisting of cotton seed residue; burnt cotton seed; mulberry leaves; rice husk or straw; oat husk or straw; sugarcane bagasse; leaves or stalk and bunch of banana trees; coffee grounds, denatured seeds, coffee husk or straw; cocoa skin and fruits; lemon balm; fat grass; guinea grass; jaguará grass; buckwheat husk; barley bagasse, husk or straw; powdered leather; poultry, cattle, horse, sheep or pig manure; eucalyptus waste; yeast extract; wheat straw, bran or flour; potato starch; straw or pigeon pea seed; bean straw; tobacco residue; Bahia or silk grass; orange pomace; cassava leaves; straw or corn cob; chicken feathers; fern; dried blood; sawdust; litter or cake made of cotton, peanuts, linseed, castor beans, soybeans or sugarcane mills;
b) Nutrients are added to the mixture;
(c) The prepared mixture is packed in containers and then sterilized or pasteurized;
II. FIRST GROWTH
d) In a microbiologically controlled environment, the fungus is added to the mixture, and the fungus is selected from the group consisting of: *Ganoderma lucidum, Ganoderma neo-japonicium, Trametes versicolor and Pleurotus ostreatus;*
e) The inoculated substrates are stored in cultivation rooms with controlled temperature and humidity for 3 to 20 days;
III. SECOND GROWTH
f) At the end of the first growth of phase II, the fungal biocomposite blocks are formed, broken and new nutrients are added to the mixture;
g) The material is packed in molds with the desired format and placed in cultivation rooms, completing the growth in 1 to 4 days;
IV. THIRD GROWTH
h) The material is removed from the mold and kept in an environment with controlled temperature, humidity and CO2 content, for 1 to 2 days;
V. FINAL PROCESSING
i) At the end of growth, the fungal biocomposite is heated to inactivate the fungus and it is dried.

2. PROCESS, according to claim 1, **characterized by** the fact that in stage a) the lignocellulosic materials are sawdust and harvest, buckwheat husks (moorish), rice husks, corn residue, soybean residue and sugarcane bagasse; and each lignocellulosic material has a specific composition.

3. PROCESS, according to any of claims 1 or 2, **characterized by** in step a) the lignocellulosic materials being crushed and sieved for size standardization (granulometry); Particles of lignocellulosic materials can be in two main forms: fiber and granule, as well as a mixture depending on the mixture used.

4. PROCESS, according to claim 1, **characterized by** the use of wheat bran, calcium carbonate, shell limestone and water as nutrients in step b, being added alone or together.

5. PROCESS, according to claim 1, **characterized by** the fact that in step c) the prepared mixture is packed in plastic, glass or metal containers.

6. PROCESS, according to claims 1 and 5, **characterized by** sterilization occurring at 121 °C for 90 minutes in step c.

7. PROCESS, according to claims 1 and 5, **characterized by** pasteurization at 70 to 80 °C for 10 to 12 hours and cooled to a maximum temperature of 35 °C in step c.

8. PROCESS, according to claim 1, **characterized by** the fact that in step e) the inoculated substrates are stored at a temperature of 20 to 33 °C; and the relative humidity of the air is at 60 to 80%.

9. PROCESS, according to claim 8, **characterized by** the fact that in step e) the inoculated substrates are stored at a temperature of 25 °C and the relative humidity of the air is at 70%.

10. PROCESS, according to claim 1, **characterized by** adding 5 to 15% of nutrient composed of a mixture composed of 74% wheat flour to the biocomposite in relation to the wet weight; 7.4% gypsum; 7.4% shell limestone; 7.4% cellulose; 3.8% lignin.

11. PROCESS, according to claim 1, **characterized by** adding 3 to 9% of nutrient composed of a mixture composed of 74% wheat flour to the biocomposite in relation to the wet weight; 7.4% gypsum; 7.4% shell limestone; 7.4% cellulose.

12. PROCESS, according to claim 1, **characterized by** the fact that in stage g) the molds are in the cultivation room at a temperature of 20 to 33 °C; humidity from 55 to 100% and CO2 from 500-50,000ppm.

13. PROCESS, according to claim 12, **characterized by** the temperature being 25 °C and the humidity 75% in step g).

14. PROCESS, according to claim 12, **characterized by** the temperature being 25 °C and the humidity 96% in step g).

15. PROCESS, according to claim 1, **characterized by** the material being kept at a temperature of 20 to 33 °C in step h); humidity from 75 to 95%; and the CO2 content in the range of 500-10,000ppm.

16. PROCESS, according to claim 15, **characterized by** the material being kept at a temperature of 25 °C, with humidity changed to 95% and the CO2 content reduced to 500-10,000ppm.

17. PROCESS, according to claim 1, **characterized by** in step i) at the end of growth, the fungal biocomposite is heated to at least 24 °C for at least 24 hours; and dry until reaching 40% of the initial weight value after the procedure.

18. FUNGAL BIOCOMPOSITES **characterized by** being obtainable from the process as defined in any of the claims 1 to 17.

19. USE OF FUNGAL BIOCOMPOSITES as defined in claim 18 **characterized by** being for the manufacture of solutions for the replacement of materials of non-renewable origin in the areas of civil construction and acoustics, protective packaging and architecture/interior design.

20. USE, according to claim 19, **characterized by** being for the manufacture of acoustic plates.
